# EUROPEAN PATENT SPECIFICATION

(11) **EP 3 353 276 B1**
(45) Date of publication and mention of the grant of the patent: **04.09.2019**
(21) Application number: 16777605.3
(22) Date of filing: 23.09.2016
(51) Int. Cl.: C12M 1/12, C12N 5/00

(54) **CELL CULTURE SUPPORT**
ZELLKULTURTRÄGER
SUPPORT DE CULTURE CELLULAIRE

(30) Priority: 23.09.2015 EP 15186375
(43) Date of publication of application: 01.08.2018
(73) Proprietor: Asociación Centro de Investigación Cooperativa en Biomateriales - CIC biomaGUNE, 20014 San Sebastián (ES)
(72) Inventor: LIZ-MARZÁN, Luis Manuel, 20009 San Sebastián (ES); GINER CASARES, Juan José, 20009 San Sebastián (ES); HENRIKSEN-LACEY, Malou, 20009 San Sebastián (ES); JOHNSON, Alexander, 20014 San Sebastián (ES)
(74) Representative: ZBM Patents - Zea, Barlocci & Markvardsen
(86) International application number: PCT/EP2016/072722
(87) International publication number: WO 2017/050984

(56) References cited:
- CHRISTINA ROSMAN ET AL: "Mammalian cell growth on gold nanoparticle-decorated substrates is influenced by the nanoparticle coating", BEILSTEIN JOURNAL OF NANOTECHNOLOGY, vol. 5, 24 December 2014 (2014-12-24), pages 2479-2488, XP055256253, DOI: 10.3762/bjnano.5.257
- ZHU M.: "Micropatterning thermoplasmonic gold nanoarrays to manipulate cell adhesion", ACS NANO, vol. 6, 2012, pages 7227-7233, XP002755211, cited in the application
- RAJA G RAYAVARAPU ET AL: "In vitro toxicity studies of polymer-coated gold nanorods", NANOTECHNOLOGY, IOP, BRISTOL, GB, vol. 21, no. 14, 9 April 2010 (2010-04-09) , page 145101, XP020174740, ISSN: 0957-4484
- PEREZ-JUSTE J ET AL: "ELECTRIC-FIELD-DIRECTED GROWTH OF GOLD NANORODS IN AQUEOUS SURFACTANT SOLUTIONS", ADVANCED FUNCTIONAL MATERIALS, WILEY - V C H VERLAG GMBH & CO. KGAA, DE, vol. 14, no. 6, 1 June 2004 (2004-06-01), pages 571-579, XP001196380, ISSN: 1616-301X, DOI: 10.1002/ADFM.200305068
- WEN LI ET AL: "Near-Infrared- and pH-Responsive System for Reversible Cell Adhesion using Graphene/Gold Nanorods Functionalized with i-Motif DNA", ANGEWANDTE CHEMIE INTERNATIONAL EDITION, vol. 52, no. 26, 24 June 2013 (2013-06-24) , pages 6726-6730, XP055256330, DE ISSN: 1433-7851, DOI: 10.1002/anie.201302048
- THEOBALD LOHMÜLLER ET AL: "Supported Membranes Embedded with Fixed Arrays of Gold Nanoparticles", NANO LETTERS, vol. 11, no. 11, 9 November 2011 (2011-11-09), pages 4912-4918, XP055322064, US ISSN: 1530-6984, DOI: 10.1021/nl202847t

## Description

The present invention relates to a cell-culture support comprising gold nanoparticles for controlled and non-invasive cell detachment of adherent cells. It also relates to a cell culture device comprising the gold nanoparticle support, a method for producing said support and its uses. Multiple technical fields can benefit from it, such as cell biochemistry, stem cell research, tissue engineering and the industrial production of cell-based therapeutics.

### BACKGROUND ART

Cell division, differentiation and behavior are controlled by many stimuli coming from the exterior of the cell. One of the most potent stimuli known in cell biology is the attachment of the cell to a physical support. The way the cell interacts with the basal membrane (or a physical surrogate) and with its neighboring cells has a profound impact on its development, differentiation, rate of division and ultimate fate. Thus, it has been recognized for many years that the efficient control of cell attachment and detachment can have many applications in a myriad of cell-based applications.

Because efficient cell growth of adherent cells implies the use of a physical support for the cells to attach to, grow and differentiate properly, one of the key steps in cell production is their detachment from their anchoring surface. This apparently simple process step is of utmost relevance in cell culture as the cells can be very easily damaged during the process and this step is commonly repeated several times in the course of a cell culture process. An efficient cell detachment technology must ensure not only that the cells are not destroyed or damaged but also that their biochemical and biophysical properties are left intact.

Applying mechanical force to drive cell detachment (cell scraping) is one of the techniques currently in use. Although it does not imply changes in the concentration of bioactive substances in the medium, it has the disadvantage of being too abrasive, causing cell damage in most cases. Moreover, from an industrial point of view, it is a highly inefficient process and difficult to automate. An alternative to this mechanical strategy is the use of digestive enzymes such as trypsin. These proteolytic enzymes disrupt the interaction between adherent cells and the cell culture support by digesting cell membrane components used by the cells for anchorage, effectively driving their detachment. However, resorting to hydrolyzing enzymes requires the use of tedious multi-step procedures implying many reagents and it often also results in cellular damage.

Of note, the cell detachment methods described above pose an important risk of contamination of cell-derived products due to the possibility of external contaminants entering the cell culture device during the detachment step.

In order to overcome the limitations cited above, many different micro-structured supports have been developed in the art with the goal of allowing easy, harmless and non-invasive cell detachment. One of the most promising strategies is based on surface materials that change their adhesive properties when exposed to a certain stimuli such as electro, thermal or photochemical stimulation. They have been called "responsive substrates" or "responsive supports" in this technical field. An especially interesting option is the activation via exposure to radiation: the goal in this case is to develop surface materials that foster the adherence and growth of cells and tissues, but which change their nature when irradiated so that they enable efficient cell detachment.

Among the materials found in the art, degradable polymers are amongst the most popular. For instance, laser-photodegradable polyketals and polyacetals have been described which can be successfully used for cell adhesion control (Pasparakis G., et.al. "Laser-induced cell detachment and patterning with photodegradable polymer substrates" Angew. Chem. Int. Ed. 2011, vol. 50, pp. 4142-4145). Carbon nanotubes which change properties when exposed to near Infrared laser have also been described for cell detachment purposes (Sada T., et.al. "Near-IR laser-triggered target cell collection using a carbon nanotube-based cell-culture substrate" ACS Nano 2011, vol. 6, pp. 4414-4421).

Remarkably, one of the most promising substrate types is the one based on gold nanoparticles. These substrate types are especially well suited for cell adhesion, division and adherence control because of their high biocompatibility. A number of references are found in the art describing applications of gold nanoparticles for the radiation-controlled release of cells from a surface.

Kolesnikova TA, et.al. "Laser-induced cell detachment, patterning, and regrowth on gold nanoparticle functionalized surfaces" ACS nano 2012, vol. 6, pp. 9585-9595, describes the use of nanoengineered gold nanoparticle functionalized (AuNP) surfaces for targeted cell detachment. The nanoparticles are uniformly distributed over a glass support, featuring sizes of 8-10nm, a thickness of 15nm and spherical shapes, devoid of anisotropy. They are capable of absorbing light in the visible wavelength (around 532nm.) which causes a plasmon resonance effect, in turn causing the generation of reactive oxygen species (ROS) which are ultimately responsible for cell detachment. In this reference, it was found that the effectiveness in cell detachment depends on a series of variables such as the age of the cells, the laser power density, the exposure time and also the patterning of the gold nanoparticles. Although the efficiency of cell detachment reported seems to be promising, the authors conclude that the most probable mechanism for cell detachment is the damage to the cell membrane due to the presence of the ROS generated. It is concluded that substrates capable of working with more biologically friendly wavelengths such as near-infrared would be more desirable. Besides, the formation of ROS, a chemical entity that diffuses in the liquid medium surrounding adhered cells, prevents the selective detachment of chosen cells by focused irradiation of selected surface areas. An additional drawback of the chemical modification of culture medium composition or adhesion surface properties is the impossibility of cell reattachment once the detachment stimuli has ceased.

Zhu M., et.al. "Micropatterning thermoplasmonic gold nanoarrays to manipulate cell adhesion" ACS Nano 2012, vol. 6, pp. 7227-7233, also describes the use of surface-immobilized nanoengineered gold nanoparticles for controlled cell attachment and detachment. The nanoparticles are deposited on glass by block-copolymer micellar lithography (BCML) giving particle sizes of up to 32nm., with inter-particle distances ranging from 25nm to 250nm, and no anisotropy. The plasmon band intensity of the final particles is centered around 530 nm, which means the nanoparticles operate within the visible light wavelengths. The authors also conclude that, although the technology developed seems to be effective at modulating cell adhesion, it would be advantageous to design gold nanostructures that could absorb wavelengths within the tissue transparency window, such as near-infrared.

Clearly, there is still the need to find alternative technologies for the efficient, harmless, reversible, selective and non-invasive detachment of adherent cells.

### SUMMARY OF THE INVENTION

Inventors have devised a structured gold nanoparticle support that is very efficient as a support for cell and tissue culture, and that operates as a responsive substrate due to a plasmonic effect when irradiated in the near-infrared (NIR) wavelength. Surprisingly, the structured gold nanoparticle support is capable of working in the NIR wavelength even with the interposition of a film layer between the cell culture support and the light source.

Remarkably, this plasmonic cell-culture support allows easy and convenient detachment of growing cells when stimulated with electromagnetic radiation in the near-infrared. This detachment step can be followed, if necessary, by the re-attachment of the cells (to the same sections of the support where they were previously attached) upon extinction of the electromagnetic radiation. The support is very versatile, enabling the growth of many different cell types, is highly biocompatible, and does not *a priori* require any further chemical or biochemical functionalization. Thus, this plasmonic gold nanoparticle support can be used in a wide range of industrial applications.

However, when needed, the support can be easily functionalized with any thiolated molecule such as for instance thiolated peptides or antibodies. These can be used on top of the gold nanoparticles, allowing the fixation of receptor-expressing cells. Thus, for instance, if the thiolated functionalized peptide is a prototypical cRGD (cyclic-Arginine-Glycine-Aspartate) peptide, the gold nanostructured support can be functionalized with the thiolated peptide and used as a growing support for cells expressing the cRGD-interacting receptors (integrins). Although one of the most convenient chemical functionalizing groups would be a thiol, other chemical groups would be equally apt such as amines and their derivatives.

Very remarkably, this gold nanostructured support displays a maximum detachment efficiency when irradiated with a NIR energy source, its production does not imply the tedious use of any reagents in a multi-step process, and cells harvested by NIR irradiation of this support are characterized by a surprisingly high cell viability. Of note, the irradiation with the NIR wavelength does not cause the formation of ROS, and the most probable cause of detachment is a photothermal effect at the cell-substrate interface due to a plasmon resonance effect.

This last point is a crucial difference with respect to what is found in the prior art (Kolesnikova, *vide supra*), as it enables the selective detachment of cells located only on certain portions of the surface, those where the beam of light impacts, and not in neighboring areas of the support. When detachment is driven by the presence of ROS, cell detachment cannot be confined only to the areas where the beam of light impacts, as ROS diffuse to surrounding areas driving detachment on areas not impacted by the beam.

Further, the support is non-immunogenic, and has a minimum influence on the morphology of the detached cells. In fact, as the data shown below clearly demonstrate, it is effective at detaching not only single cells but also whole cell sheets. Depending on the cell type, the support can even enable the detachment of multiple layers of cells.

An additional feature of the cell culture supports of the invention is that the Surface Enhanced Raman Spectroscopy (SERS) signal obtained from them is intensified by the adhesion of cells to the surface said supports. This characteristic is exploited to monitor cell propagation and confluence on the culture support by probing it with a SERS probe. This way of monitoring cell growth avoids the tedious and sometimes impractical detection of cell growth by direct optical observation.

The nanoparticles comprised in the nanostructured plasmonic support of the invention resemble, under the Scanning Electron Microscope (SEM), an anisotropic aggregate or cluster of several anisotropic particles randomly organized in individual particles. These grape cluster-like particles, which are between 2 and 30 nm apart from one another, are of various sizes: from a few nanometers to a couple of microns. Inventors have realized the relevance of all this features in the technical advantages of the invention. This geometry is the result of the manufacturing process employed to produce the supports. Remarkably, the plasmon band of the supports of the invention is very broad, presenting a maximum at NIR wavelengths but absorbing at shorter wavelengths too, allowing, for example, for detection of bound substances applying SERS at wavelengths different from those used for cell detachment.

The gold nanostructured plasmonic support of the invention thus covers the long felt need for biocompatible surfaces that not only work at a biologically friendlier wavelength, but also allow for the recovery of nearly intact cells in a very efficient, harmless, reversible, selective and non-invasive manner. This goal has been achieved thanks to the finding of the specific features that the nanoparticles of gold must have to be capable of generating a plasmon effect that drives cell-detachment via heat formation when irradiated with a NIR source (approximately 780-2500 nm). These features are the specific particle size, anisotropy, and inter-particle distance, together with the partial embedding of the nanoparticles in the support and the grape cluster-like shape. As it is illustrated in the Examples in a comparative way, the supports comprising nanoparticles of gold previously disclosed in the art (such as those of Zhu M., et.al. (*vide supra*)), do not yield good results when they are used in the NIR range. Consequently the finding of the specific conditions needed to work with NIR radiation are considered a contribution to the art.

Thus, a first aspect of the present invention is an adherent cell-culture support comprising gold nanoparticles on the surface where the cells attach to, wherein the size of the nanoparticles is from 5 nm to 2 microns, the distance between the nanoparticles is from 2 nm to 30 nm, and the anisotropy aspect ratio is greater than 1, wherein the nanoparticles are adhered to the support, the nanoparticles are totally or partially embedded in the support and the shape of the nanoparticles is a grape cluster shape.

A second aspect of the present invention is a cell-culture device comprising the adherent cell-culture support comprising gold nanoparticles of the first aspect of the invention.

A third aspect of the present invention is a cell culture bioreactor comprising the cell-culture device of the second aspect of the invention, and further comprising means to irradiate the cell-culture device of the second aspect of the invention with a Near Infrared Light (NIR).

A fourth aspect of the present invention is a process for producing an adherent cell-culture support comprising gold nanoparticles on the surface where the cells attach to, comprising the steps of: a) Deposition of inverse micelles of a copolymer, comprising gold ions in the interior, onto the surface; b) The result of step a) is treated with oxygen plasma for a period of from 2 to 4 minutes; c) The result of step b) is treated twice with a solution of gold ions, a reducing agent and a surfactant, for at least 30 minutes each treatment; d) The result of step c) is treated with oxygen plasma for a period of at least 10 minutes.

A fifth aspect of the present invention is an adherent cell-culture support comprising gold nanoparticles on the surface where the cells attach to, obtainable by the process of the fourth aspect of the invention.

A sixth aspect of the present invention is a method for culturing cells that comprises a step of seeding cells on the adherent cell-culture support of the first and fifth aspects of the invention. The method has a range of potential industrial applications in which many different cell types can be conveniently grown and retrieved by a minimally invasive technique, one which additionally allows maximum recovery efficiency with the application of a relatively mild and harmless energy source.

### BRIEF DESCRIPTION OF THE DRAWINGS

FIG.1. Scanning Electron Microscopy (SEM) pictures of the Au seeds deposited on the surface of a glass after the short treatment with oxygen plasma. (scale bar is 300 nm).
FIG.2. SEM picture of the plasmonic support after the chemical growth step and cleaning with a long treatment of oxygen plasma. A) Scale bar is 500 nm B) Scale bar is 1 micron C) Scale bar is 3 microns D) Scale bar is 10 microns
FIG.3. Visible - Near Infrared (Vis-NIR) spectra of plasmonic substrates before (dashed line) and after (solid line) the growth step. Y is Absorbance and X is Wavelength in nm.
FIG. 4. Cell morphology of cells grown on various substrates. From top to bottom: HeLa, A549, HUVEC and J774. Left column: A.R. is aspect ratio of the cells (defined as the ratio between the longest and shortest dimensions). Right column: C.A. is area per cell (in square micrometers). Key is as follows: Bare glass slides (G), bare plasmonic substrates (Au), and c-RGD functionalized plasmonic substrates (RGD).
FIG. 5. Cell detachment from various substrates by NIR radiation. From top to bottom: HeLa, A549, HUVEC, J774. Keys are as follows: percentage cell detachment (C.D.%), substrate (S), bare glass slides (G), bare plasmonic substrates (Au), and c-RGD functionalized plasmonic substrates (RGD).
FIG. 6. Detachment of a 3T3 fibroblast cell sheet. Microscopy images of a folded 3T3 NIH fibroblasts sheet grown on a bare plasmonic substrate of the invention and subsequently detached by irradiation with NIR light.
FIG.7. Cell survival rate (C.S.%), measured with the MTT cell growth assay, after NIR-mediated cell detachment from plasmonic substrates. HeLa, A549, HUVEC, and J774 cells were studied on both bare plasmonic substrates (solid bars) and c-RGD functionalized plasmonic substrates (columns with diagonal patterned lines).

### DETAILED DESCRIPTION OF THE INVENTION

For the sake of understanding, the following definitions are included and expected to be applied throughout description, claims and drawings.

The terms "substrate" and "support" are herein used interchangeably, so that cell-culture support and cell-culture substrate can be taken as synonyms, as well as plasmonic cell-culture support and plasmonic cell-culture substrate.

The term "bare plasmonic substrate" is considered here as a synonym of "bare plasmonic support", and as used herein refers to the adherent cell-culture support of the invention when it is not coupled to any chemically functionalized molecule featuring chemical groups capable of enabling the attachment to a gold surface.

The term "gold nanoparticle" as used herein refers to a nanoparticle made of gold. A nanoparticle is a particle that has a size between 5 and 2000 nanometers, and is defined as a small object that behaves as a whole unit with respect to its transport and properties.

As used herein, the term "size" refers to a characteristic physical dimension. For example, in the case of a nanoparticle that is substantially spherical, the size of the nanoparticle corresponds to the diameter of the nanoparticle. In the case of a nanoparticle that is substantially rod-shaped with a substantially circular cross-section, such as a nanowire or a nanotube, the size of the nanoparticle corresponds to the diameter of the cross-section of the nanoparticle. In the case of a nanoparticle that has a spaghetti-like shape, the size of the nanoparticle is the size of its section, not the size of its overall length. In the case of a nanoparticle that is substantially cube-shaped or rectangular, such as a nanocube, a nanobox, or a nanorectangle, the size of the nanoparticle corresponds to the maximum edge length. In the case of an irregular particle, the size of the particle is the maximum length of the particle as projected in any dimension. When referring to a set of nanoparticles as being of a particular size, it is contemplated that the set of nanoparticles can have a distribution of sizes around the specified size. Thus, as used herein, the size of a set of nanoparticles refers to a mode of a distribution of sizes, such as a peak size of the distribution of sizes.

Related to this, the methods for measuring particle size and inter-particle distance used herein were all based on Scanning Electron Microscopy (SEM).

Some materials do not have the same properties or the same sizes or shapes in all directions. For example, a material can be very resistant in one axis and less resistant in second axis. A second material can be long on one axis and short on a second axis. Some materials display a very uniform range of sizes, but with varying shapes, etc. This property of being dependent on the directionality is called anisotropy (as opposed to isotropy, which means that the properties, sizes or shapes are the same, irrespective of the spatial direction). As an example, the "anisotropy aspect ratio" of a bidimensional structure such as a rectangle is the ratio of its longer side to its shorter side (the aspect ratio can be expressed as the relationship of the two numbers, or simply the quotient). For instance if the longer side of a rectangle is 2 and the shorter side is 1, then the "anisotropy aspect ratio" could be expressed as 2:1, or simply 2. The aspect ratio of a square or a sphere is 1:1, or simply 1.

The term "adsorption" of a gold nanoparticle to a surface as used herein refers to the adhesion of the nanoparticle to said surface. The process creates a layer of gold nanoparticles on the surface. The adhesion of the nanoparticles to the surface of the support implies the partial insertion of the nanoparticles on the nanoholes found on the surface, so that the particles are effectively embedded (at least partially) in them.

The term "grape cluster shape" concerning a gold nanoparticle, refers to a nanoparticle of gold that, irrespective of its size, has a shape that resembles that of a cluster of grapes, that is, it is structured in a way that several roundish elements are grouped together to form the whole of the nanoparticle.

The term "thiolated molecule" as used herein refers to any molecule to which one or more thiol groups (R-SH) have been chemically attached.

The term "cellular transfection" as used herein refers to the process of introducing foreign genetic material (such as DNA or RNA) into a host cell by any non-viral means such as electroporation.

The term "inverse micelle" as used herein refers to a micelle in which the hydrophilic groups of the molecules forming the micelle are sequestered in the interior or core (capable of accommodating gold ions) and the hydrophobic groups of the molecules forming the micelle extend away from this core to the exterior, exposing themselves to a non-polar solvent.

The term "PS-b-P2VP" is a shorter version for block-copolymer poly(styrene-b-2-vinylpyridine). This is a block copolymer forming inverse micelles in solution of an organic solvent containing the Au ions in the core. In our experiments the organic solvent is toluene, although other organic solvents can also be used, e. g., o-xylene. The molecular weight of the PS-b-P2VP is 162.000 g/mol, with Mw(PS) = 110.000 g/mol and Mw(P2VP) = 52.000 g/mol.

Other PS-b-P2VP with different values of molecular weights in the range of 20.000 to 400.000 can be used.

The term "dip-coating" as used herein, refers to a process based on coating of a surface by immersion. A thin film is applied onto a substrate which is flat or cylindrical. It usually entails 5 steps: i) immersion (the substrate is placed in the solution of the material with which it is to be covered, at a constant speed); ii) start (the substrate has remained in the solution for a certain period of time); iii) deposition (the thin film is deposited onto the substrate, while it is taken out of the solution at a constant speed) iv) draining (the excess liquid of the solution is drained from the surface); v) evaporation (the solvent is evaporated).

As mentioned above, a first aspect of the present invention is an adherent cell-culture support comprising gold nanoparticles on the surface where the cells attach to, wherein the size of the nanoparticles is from 5 nm to 2 microns, the distance between the nanoparticles is from 2nm to 30 nm, and the anisotropy aspect ratio is greater than 1.

In a particular embodiment of the first aspect of the invention, the size of the nanoparticles is from 10 nm to 1 micron, the distance between the nanoparticles is from 2 to 30 nm, and the anisotropy aspect ratio is greater than 1.01.

In another particular embodiment of the first aspect of the invention, the anisotropy aspect ratio is equal to or greater than 1.01 and equal to or lower than 100. In another particular embodiment of the first aspect of the invention, the anisotropy aspect ratio is equal to or greater than 1.1 and equal to or lower than 50.

In a particular embodiment of the first aspect of the invention the size of the nanoparticles is from 10 nm to 1 micron, the distance between the nanoparticles is from 2 to 30 nm, and the anisotropy aspect ratio is greater than 1.

In a particular embodiment of the first aspect of the invention the size of the nanoparticles is from 20 nm to 500nm, the distance between the nanoparticles is from 2 to 30 nm, and the anisotropy aspect ratio is greater than 1.

In a particular embodiment of the first aspect of the invention the size of the nanoparticles is from 30 nm to 500nm, the distance between the nanoparticles is from 2 to 30 nm, and the anisotropy aspect ratio is greater than 1.

In a particular embodiment of the first aspect of the invention the size of the nanoparticles is from 30 nm to 50nm, the distance between the nanoparticles is from 1 to 10 nm, and the anisotropy aspect ratio is greater than 1.

In a particular embodiment of the first aspect of the invention the size of the nanoparticles is from 35 nm to 500nm, the distance between the nanoparticles is from 2 to 30 nm, and the anisotropy aspect ratio is greater than 1.

In a particular embodiment of the first aspect of the invention the size of the nanoparticles is from 33 nm to 1000nm, the distance between the nanoparticles is from 1 to 24 nm, and the anisotropy aspect ratio is greater than 1.

In a particular embodiment of the first aspect of the invention the size of the nanoparticles is from 33 nm to 500nm, the distance between the nanoparticles is from 1 to 24 nm, and the anisotropy aspect ratio is greater than 1.

In a particular embodiment of the first aspect of the invention the size of the nanoparticles is from 35 nm to 500nm, the distance between the nanoparticles is from 2 to 20 nm, and the anisotropy aspect ratio is greater than 1.

In a particular embodiment of the first aspect of the invention the size of the nanoparticles is from 50 nm to 500nm, the distance between the nanoparticles is from 1 to 20 nm, and the anisotropy aspect ratio is greater than 1.

In a particular embodiment of the first aspect of the invention the size of the nanoparticles is from 50 nm to 500nm, the distance between the nanoparticles is from 1 to 10 nm, and the anisotropy aspect ratio is greater than 1.

In another particular embodiment of the first aspect of the invention the nanoparticles are adsorbed onto the surface. This means that the nanoparticles are adhered to the surface. As stated above, the adhesion can in certain instances also imply their total or partial embedding into the holes found on the surface.

According to the invention the nanoparticles are partially inserted, i.e. embedded, on the surface of the support due to the plasma treatment.

According to the invention, the shape of the particles is a grape cluster shape.

In another particular embodiment of the first aspect of the invention the support is a glass support.

In another particular embodiment of the first aspect of the invention the support is an organic polymer support.

In another particular embodiment of the first aspect of the invention the support is a polystyrene, polycarbonate or polyethylene terephthalate support.

In another particular embodiment of the first aspect of the invention the support further comprises thiolated molecules bound to the nanoparticles via their sulphur atoms. The thiolated molecules may be covalently bound to the gold nanoparticles of the support via their thiol groups. In this way, a range of molecules can be prepared so that they can be fixed to the adherent cell-culture support comprising the structured gold nanoparticles.

In another particular embodiment of the first aspect of the invention the support further comprises molecules such as proteins, peptides and nucleic acids, with a range of chemical functionalities such as amines or derivatives through which the molecules are anchored to the gold nanoparticles of the adherent cell-culture support..

In another particular embodiment of the first aspect of the invention the support further comprises macromolecules bound to the gold nanoparticles of the cell-culture support by non-specific binding, such as albumin or other proteins.

In another particular embodiment of the first aspect of the invention the molecules are selected from the group consisting of proteins, peptides, organic molecules, nucleic acids and their thiolated counterparts.

In another particular embodiment of the first aspect of the invention the thiolated molecules are peptides.

In another particular embodiment of the first aspect of the invention the thiolated peptides are peptides capable of binding to an integrin receptor.

In another particular embodiment of the first aspect of the invention the thiolated peptide is a cRGD peptide.

As mentioned above, a second aspect of the invention is a cell-culture device comprising the adherent cell-culture support of the first aspect of the invention.

In a particular embodiment the cell-culture device of the second aspect of the present invention further comprises a switchable light source that, upon activation emits light in the NIR wavelength directed towards the cell-culture support.

In another particular embodiment of the cell-culture device of the second aspect of the invention, the cell culture device further comprises a Surface Enhanced Raman Spectroscopy (SERS) probe.

In a particular embodiment of the second aspect of the invention, the cell-culture device is a bioreactor. In a particular embodiment of the second aspect of the invention, the culture device is a bioreactor that comprises a light source that upon activation emits light in the NIR wavelength directed towards the cell-culture support.

As mentioned above, a fourth aspect of the present invention is a process for producing an adherent cell-culture support comprising gold nanoparticles on the surface where the cells attach to, comprising the steps of: a) Deposition of inverse micelles of a copolymer, comprising gold ions in the interior, onto the surface; b) The result of step a) is treated with oxygen plasma for a period of from 2 to 4 minutes; c) The result of step b) is treated twice with a solution of gold ions, a reducing agent and a surfactant, for at least 30 minutes each treatment; d) The result of step c) is treated with oxygen plasma for a period of at least 10 minutes.

In a particular embodiment of the fourth aspect of the invention, the deposition in step a) is a deposition by dip-coating.

In another particular embodiment of the fourth aspect of the invention, the deposition in step a) is a deposition by a technique selected from the group consisting of spin coating, Doctor blade and deposition by evaporation.

In a particular embodiment of the fourth aspect of the invention, the copolymer in step a) is block-copolymer poly(styrene-b-2-vinylpyridine) (PS-b-P2VP).

In another particular embodiment of the fourth aspect of the invention, the copolymer in step a) is selected from the group consisting of polystyrene, polyethylene glycol and polyvinylpyrrolidone, or a combination thereof.

In a particular embodiment of the fourth aspect of the invention, in step c) the reducing agent is ascorbic acid.

In another particular embodiment of the fourth aspect of the invention, the reducing agent in step c) is selected from the group consisting of sodium borohydride and potassium triethylborohydride.

In a particular embodiment of the fourth aspect of the invention, in step c) the surfactant is cetyl trimethylammonium bromide (CTAB).

In a particular embodiment of the fourth aspect of the invention, in step c) the surfactant is Triton. In another embodiment, in step c) the surfactant is Triton-X100.

In a particular embodiment of the fourth aspect of the invention, in step c) the reducing agent is ascorbic acid and the surfactant is cetyl trimethylammonium bromide (CTAB).

In a particular embodiment of the fourth aspect of the invention, the copolymer in step a) is block-copolymer poly(styrene-b-2-vinylpyridine) and the reducing agent in step c) is ascorbic acid and the surfactant is cetyl trimethylammonium bromide (CTAB).

In a particular embodiment of the fourth aspect of the invention, the concentrations of the gold ions, the reducing agent and the surfactant in step c) are 8-800 µM, 0.1-10 mM, and 2-1000 mM, respectively.

In a particular embodiment of the fourth aspect of the invention, the concentrations of the gold ion HAuCl4, the reducing agent ascorbic acid and the surfactant CTAB in step c) are 8-800 µM, 0.1-10 mM, and 2-1000 mM, respectively.

As also stated above, a fifth aspect of the present invention is an adherent cell-culture support comprising gold nanoparticles obtainable by the process of the fourth aspect of the invention.

According to the fifth aspect of the invention, the adherent cell-culture support obtainable by the process of the fourth aspect of the invention comprises gold nanoparticles on the surface where the cells attach to, wherein the size of the nanoparticles is from 5 nm to 2 microns, the distance between the nanoparticles is from 2 nm to 30 nm, and the anisotropy aspect ratio is greater than 1, wherein the nanoparticles are absorbed onto the surface of the support and are partially or totally embedded on the support.

In another particular embodiment of the fifth aspect of the invention, the adherent cell-culture support obtainable by the process of the fourth aspect of the invention comprises gold nanoparticles wherein the size of the nanoparticles is from 30 nm to 500 nm and the distance between the nanoparticles is from 1 to 24 nm.

According to the fifth aspect of the invention, the adherent cell-culture support obtainable by the process of the fourth aspect of the invention comprises gold nanoparticles wherein the shape of the particles is a grape-cluster shape and whose anisotropy aspect ratio greater than 1.

As also stated above, a sixth aspect of the present invention is a method for culturing cells that comprises a step of seeding cells on the adherent cell-culture support of the first and fifth aspect of the invention. The seeding of the cells, which consists of placing them in contact with the support for a fixed period of time and under defined conditions, can sometimes imply their adhesion to the adherent cell-culture support, it can imply their covalent attachment to said support, or both.

In a particular embodiment of the sixth aspect of the invention, the method for culturing cells further comprises a cell detachment step comprising an irradiation step of the cell loaded culture support with Near Infrared Light (NIR).

In a particular embodiment of the sixth aspect of the invention, the method for culturing cells further comprises a step of cell detection comprising the detection of the Surface Enhanced Raman Spectroscopy signal emitted by the cell loaded culture supports.

In a particular embodiment of the sixth aspect of the invention, the method for culturing cells further comprises a cell transformation step comprising the incubation of the detached cells with a nucleic acid. This step would enable the cellular transfection of the cells grown on the adherent cell-culture support of the first and fourth aspect of the invention.

Throughout the description and claims the word "comprise" and variations of the word, are not intended to exclude other technical features, additives, components, or steps. Furthermore, the word "comprise" and its variations encompass the term "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### A) Material and Methods

Materials. Hexadecyltrimethylammonium bromide (CTAB, >96%), hydrogen tetrachloroaurate trihydrate (HAuCl4x3H₂O, >99.9%), L-ascorbic acid, >99%), Triton X-100 (laboratory grade), Tween 20, sodium chloride (>99%), sodium phosphate (>99%), Dimethylsulfoxide (>99.9%) and ammonium hydroxide solution (30% w/w) were purchased from Aldrich. Hydrogen peroxide solution (35% w/w) and Toluene were purchased from Scharlau. Poly(styrene-b-2-vinyl pyridine) (PS₁₀₅₀-P2VP₄₉₅) was purchased from Polymer Source, Inc. DMEM, FBS, PS, trypsin-EDTA, Dapi, and WGA-AF647 were purchased from Invitrogen. All chemicals were used as received. Milli-Q water (resistivity 18.2MΩ cm at 25 °C) was used in all experiments. Picodent twinsil (Picodent, Wipperfürth, Germany) was used to glue the cut-off eppendorf tubes to the plasmonic substrates. HeLa cells and 3T3 NIH fibroblasts were kindly given by Charles Lawrie and Ander Izeta, respectively (Biodonostia, Donostia-San Sebastián, Spain). A549, HUVEC, and J774 cells were kindly given by Sergio Moya, Marco Marradi, and Juan Mareque, respectively (CIC biomaGUNE).

Methods. All glassware was washed with aqua regia, rinsed with water, sonicated three times for 3 min with Milli-Q water, and dried before use. Oxygen plasma treatment was performed in a Diener plasma chamber (Diener, Ebhausen, Germany). Optical extinction spectra were recorded using an Agilent 8453 UV-vis diode-array spectrophotometer and a Cary 5000 UV-vis-NIR spectrophotometer (Varian, Inc). SEM (Scanning Electron Microscopy) images were obtained using an ESEM (Environmental Scanning Electron Microscopy) Quanta250 FEG (FEI, The Netherlands). XPS (X-ray photoelectron spectroscopy) experiments were performed in a SPECS Sage HR 100 spectrometer with a non monochromatic X ray source (Magnesium Kα line of 1253.6 eV energy and a power applied of 250 W and calibrated using the 3d5/2 line of Ag with a full width at half maximum (FWHM) of 1.1 eV. An electron flood gun was used to compensate for charging during XPS data acquisition. The selected resolution for the spectra was 15 eV of Pass Energy and 0.15 eV/step. All Measurements were made in an ultra high vacuum (UHV) chamber at a pressure around 5x10e-8 mbar. Static contact angle measurements were performed with a DSA100, Krüss GmbH, (Hamburg, Germany). A Cell Observer Zeiss microscope was used to record bright field and fluorescence pictures of the cells on the substrates.

Fabrication of plasmonic substrates. Glass supports were cleaned prior to the deposition and growth of the monolayer of plasmonic nanoparticles with basic piranha solution (1:1:5 volume fraction of hydrogen peroxide solution, ammonium hydroxide solution, and water, respectively) for 2 hours at 60 °C. The fabrication of the plasmonic substrates was divided into two steps: 1) deposition of Au seeds onto the surface, and 2) growth of Au seeds into anisotropic nanoparticles.
1) Deposition of the Au seeds was based on a variation of the block copolymer micellar nanolithography (BCML) technique. Basically, a monolayer of inverse micelles of a copolymer with Au atoms contained in the core was deposited by dip-coating on the surface of the support. The dispersion of the inverse micelles was prepared as follows; i) a 5 mg/mL dispersion of the block copolymer was prepared in toluene and stirred for 24 hours, ii) 0.5 mol of Au atoms (as hydrogen tetrachloroaurate trihydrate) per each P2VP unit was added, and the dispersion was stirred for 24 hours, iii) the dispersion was let without stirring for 24 hours, iv) the dispersion was transferred to a clean container except for a few mL in the bottom, usually appearing turbid. All fabrication steps of the dispersion, as well as the storage, were performed under dark conditions. The glass supports were dip coated (using a dip coater KSV sigma 70, from KSV Instruments), with a dip speed of 26 mm/min in the BCML dispersion. Immediately after dip-coating, the substrates were treated with oxygen plasma at the following conditions: 1.47 mbar, 200 W, 4 min. At this point, the substrates were covered with a monolayer of Au nanoparticles seeds with certain coverage of copolymer..
2) Growth of Au seeds into anisotropic nanoparticles. The substrates were subjected to two growth steps by immersing the substrates into an aqueous growth solution (HAuCl4:AA:CTAB 1:12.5:50 in molar ratio).. The concentration of ascorbic acid (AA) was 1 mM. The ascorbic acid was freshly prepared and immediately used. No waiting time was allocated between the two growth steps. The substrates were rinsed with Milli-Q water and gently blow-dried with a stream of nitrogen gas. A final treatment with oxygen plasma was required with the following conditions: 1.47 mbar, 200W, 50 min. The plasmonic substrates were stored in Petri dishes.

Fabrication of plasmonic cell supports found in the art. In order to study the light absorption properties of other supports found in the prior art, we fabricated substrates according to Zhu et al.(*vide supra*). Briefly, we used a solution of (PS1056-b-P2VP495) from Polymer Source Inc. in toluene at a concentration of 5 mg/mL. The quantity of gold precursor was calculated relative to the number of P2VP units (NP2VP) with a loading parameter (L) equal to 0.5, that is, 1 molecule of HAuCl4 for 2 P2VP monomers. Hydrogen tetrachloroaurate(III) trihydrate (HAuCl4.3H2O, Sigma-Aldrich) was added to the polymer solution and stirred for 2 days in a sealed glass vessel. Glass coverslips (Carl Roth) were cleaned in a piranha solution for at least 5 h and were extensively rinsed with Milli-Q water and dried under a stream of nitrogen. Gold-loaded micellar monolayers were prepared by dip-coating a glass coverslip into the previously prepared solutions with a constant velocity equal to 24 mn·min-1. To remove the organic template and form inorganic nanoparticles, the dip-coated glass slides were exposed to oxygen plasma. A further growth of the deposited Au nanoparticles was performed by incubating the substrate for ca. 30 minutes in 10 mL of an aqueous solution containing ethanolamine (2 mM) and KAuCl4 (0.1 wt %) in 1, 2, and 3 growth steps. It was seen that in all cases, the maximum wavelength of the plasmon band was centered at ca. 530 nm, with no absorption in the NIR region (thus, it was clearly seen that the plasmonic surfaces described in the prior art could not work in the NIR region).

Biofunctionalization of the plasmonic supports with c-RGD. Cyclic-RGD (cyclo [Arg-Gly-Asp-D-Phe-Lys(Ac-SCH2CO) 1) was synthesized at a purity of 95% by Peptides International. The peptide was equipped with a thioacetyl group at the lysine residue for linking to Au surfaces. Acetyl protected cRGD, typically (7.12 mg) was deacetylated in an aqueous solution of 0.05 M hydroxylamine-HCI/0.03 M of ethylenediaminetetracetic acid, pH 7.0 for two days to yield deacetylated RGD peptide 2 (94%). The reaction was monitored by 1H-NMR and proceeded until the singlet signal at 2.3 ppm, assigned to CH3CO-S (S-thioacetyl group) disappeared. The plasmonic substrates were soaked in a solution of the c-RGD peptide with a concentration of 50 µM during 12 hours.

Cell culture on cell culture supports. HeLa, A549, J774 and 3T3 cells were grown in independent cell culture flasks (Nunc t-flasks, Thermo Scientific) containing DMEM media supplemented with 10 % FBS and 1 % Pen-Strep. HUVEC cells were grown in F12-K media supplemented with 10 % FBS, 1 % Pen-Strep, heparin (0.1 mg/ml) and endothelial cell growth fractor (ECGF; 30 ug/ml). Cells were harvested using Trypsin-EDTA, except in the case of J774 cells which were harvested using pipetting. Subsequently cells were seeded within a holder made of a cut-off 1.5 ml plastic vial (eppendorf) glued onto the cell culture supports, with or without gold/gold-RGD covering. The cells were allowed to grow and adhere to the supports for at least one night at 37 °C, 5 % CO₂ and 95 % relative humidity.

Cellular growth detection by SERS. Plasmonic substrates or cell-loaded plasmonic substrates are placed on a Renishaw Invia Raman microscope for acquiring the SERS spectra. The Renishaw Invia Raman microscope is equipped with two Peltier-cooled CCD detectors, a Leica microscope with two gratings with 1200 and 1800 lines/mm and band-pass filter optics.

A diode laser with emission wavelengths of 785 nm is used and focused onto the solid sample through a 50x objective with a NA of 0.5. The substrates are irradiated with laser powers of 0.2mW. The exposition time is set to 1 s and one scan accumulated. The characteristic Raman bands corresponding to proteins and lipid are monitored to follow the growth of cells, mainly those bands at 495-520 cm⁻¹, 1100-1140 cm⁻¹, 1300-1325 cm⁻¹. The signal is obtained only when cell-loaded plasmonic substrates are irradiated.

Cell harvest from the plasmonic substrates. Cells were cultured on the plasmonic substrates as described in a previous example and exposed to NIR irradiation using a NIR laser at 980 nm, and intensities for different periods of time according to Table 1, found below. The irradiation step was followed by a brief incubation time at 37 °C, 5 % CO₂ and 95 % relative humidity. After this incubation time, cells were detached. After detachment, the culture media with the floating cells was gently pipetted and placed in a 96-well plate. A thin layer of the culture media was left in contact with the plasmonic substrates to avoid damage of the cells and mechanically flow-directed detachment of cells.

Reversible cell detachment. Cells cultured on the plasmonic substrates are subjected to detachment by irradiation with NIR laser as described in previous examples. After cell detachment, the plasmonic substrates are incubated for ca. 4 hours at 37 °C, 5 % CO₂ and 95 % relative humidity. Then, a progressive re-attachment of the detached cells is observed by optical microscopy. After 2 additional hours, most of the detached cells are again attached to the plasmonic substrates.

Selective and localized cell detachment. The NIR laser is focused by a lens system onto a circular beam spot of ca. 1 square micrometer. By coupling the NIR laser to an optical microscope equipped with visual recording equipment and a cell culture chamber, we irradiate a given living cell while observing it with the microscope. After the typical time of detachment described in previous examples, the cell is detached. The surrounding cells are not detached or show any changes in morphology or signs of modification.

Cellular transfection. In order to assess the ability of the laser treatment to induce pores in the membrane of cells, A549 cells are chosen as a model system for the uptake of a GFP-expressing plasmid. As a positive control, cells are electroporated with the GFP-expressing plasmid and compared to the results obtained from A549 cells exposed to GFP-expressing DNA immediately post laser treatment. Cells are transfected with pcDNA3 eGFP (Addgene; plasmid #13031), a gift from Doug Golenbock, which came in agar stabs with E. coli DH5α. The plasmid contains bacterial resistance to amphicillin and their eukaryotic selection marker is G418 (Sigma Aldrich). For the electroporation procedure, A549 cells are grown in 75 cm2 flasks until reaching 80% confluence and were centrifuged at 300g for 5 minutes at 4 °C, after treatment with trypsin. The pellet is suspended in 5 ml of PBS and cell concentration is measured in a hemocytometer using Trypan blue staining. Cells are collected by centrifugation and resuspended in DMEM media without FBS or Penicillin or Streptomycin at a final concentration of 2,5 x 10⁶ cells/ml. 400 µl of cell suspension are transferred into an electroporation cuvette (BioRad) on ice. 10 µl of GFP-expressing DNA at a concentration of 1 µg/µl (determined using a Nanodrop) are added to each cuvette and the suspension is mixed gently by pipetting. The mixture is electroporated at 260 V and 1,050 µF and immediately placed on ice. Once all samples are electroporated, cells are transferred into 24-well plates dividing each 400 ul sample into 4 wells, 100 ul each. The wells already contain 900 ul media (with FBS).
For the transfection method using laser treatment, cells are cultured on the plasmonic substrates. FBS-containing media is replaced with media without FBS. The cells are subjected to the detachment procedure by irradiating with NIR light as described above. Right after the irradiation with NIR light, DNA (10 ul) is immediately added. The sample is returned to the incubator and as in the case of typical electroporation, GFP expression followed after approximately 24hrs using 470nm LED excitation and GFP filters on a Cell Observer Zeiss microscope.

Reusability of the plasmonic substrates. The cells cultured on the plasmonic substrates are subjected to detachment by irradiation with NIR laser as described previously. The detached cells are harvested and placed in a 96-well plate. The plasmonic substrates are then washed with culture media. A new set of cells is then cultured on the plasmonic substrates as described previously. The new cells display a growth rate and morphology equal to the first culture. This set of cells is detached by NIR light using the procedure described above. The efficiency of cell detachment as well as cell viability is equal to those in the first usage of the plasmonic substrate.

Flow cytometry. A549, HUVEC and HeLa cells monolayers were harvested from the cell coated culture supports by adding room temperature solution of Accutase in PBS Sigma. Cells were counted, washed with 1 % BSA/PBS and placed in cytometry tubes at 1x10⁵ cells/tube. Cells were stained with anti-αVβ3 mouse anti-human monoclonal antibody (LM609; Milipore), diluted 1/400 in 0.1% BSA/5% donkey serum/PBS and incubated in ice for 30 minutes. Cells were washed twice with BSA/PBS and re-suspended in donkey anti-mouse polyclonal IgG-AF647 (Abcam), diluted 1/2000 in BSA/donkey serum/PBS. Cells were incubated in the dark in ice for 30 minutes followed by two more BSA/PBS washes and finally re-suspended in 200 ul BSA/PBS. Cells were run on a Canto II BD flow cytometer and αVβ3 expression measured in the APC channel, collecting 5,000 cell events.

Fluorescent staining. Cells, grown on plasmonic cell-culture supports or on bare glass supports were treated with formaldehyde (2% in PBS) for 20 minutes at room temperature (RT). After washing with ice-cold PBS, cells were stained with Wheat-germ agglutinin (WGA)-Alexa Fluor 647 (1/200 in PBS) for 20 minutes at RT. Following washing with ice-cold PBS, cells were permeabilized using 0.25 % Triton X-100 (10 min, RT) and then blocked using 1 % BSA in PBST (PBS 10 mM with 0.1 % Tween-20) for 30 mins at 37 °C. Block was removed and without washing, anti- αVβ3 (LM609; Milipore) or anti-vinculin (SMP227; Abcam) were added, diluted in PBST. After 1 hr incubation at 37 °C, samples were washed with ice-cold PBS and anti-mouse IgG-AF488 added (1/500 dilution in 1 % BSA in PBS). Samples were left in the dark for 1 hr at RT, followed by washing and then by addition of actin stain (1/50 in PBS). After 20 mins at RT, samples were washed again and then stained with DAPI (1/600 in media) followed by extensive washing. Brightfield and fluorescence microscopy photos were taken on a Cell Observer Zeiss microscope using either a x20 (NA 0.8), x40 (NA 1.3) or x63 (NA 1.3) objective. Dapi, GFP and RFP filters were used to detect the cell nucleus, integrin/vinculin and plasma membrane staining, respectively. At least 4 images/format were taken.

Live cellular imaging. For experiments in which cells were subjected to laser treatment and then followed over time, no fluorescence staining was conducted but cells were simply imaged using brightfield microscopy. Cells, grown on glass coverslips were left overnight to adhere and were then imaged to obtain control pre-laser morphology and information relating to cell numbers. After applying laser treatment to cells, samples were returned to a cell culture incubator (37 °C, 5% CO₂, 95 % relative humidity) and at various time points thereafter removed and photos taken using brightfield microscopy. In certain cases cells, which had become dislodged post laser treatment, were removed and re-plated in either a 96-well plate or a 384-well plate (the later when the number of dislodged cells was very low). Between 4-8 hours post laser treatment, cell media was replaced with a solution of live and dead stains, diluted 1/1000 in staining buffer (Live/Dead cell staining kit, Abcam). Brightfield and fluorescence microscopy photos were taken 15 mins after using filters for GFP and RFP for live and dead stains respectively. A Cell Observer Zeiss microscope with a x10 or x20 objectives was used to take all images. Three to five images per/format were collected to calculate an average count.

Cell viability MTT assay. In order to determine whether cells were still metabolically active after laser treatment inventors used the MTT reagent. Cells were grown as described above; approximately 4 hrs post laser treatment dislodged cells were removed and plated in a 96-well plate. Cells which did not lift up were dislodged using trypsin-EDTA and placed in separate wells of the same 96-well plate. Control wells were also plated which comprised A) cells grown directly in the 96-well plate, and B) cells grown under the same conditions of glass/gold/gold-cRGD but without any laser treatment. In all cases the initial number of cells was the same for each cell line. The following day culture medium was removed from every well and 100ul MTT reagent (1/20 dilution in media) added. Cells were incubated for 2hrs at 37 °C followed by removal of the media and addition of 100 ul/well DMSO. Absorbance at 550nm was read using a plate reader.

Harvest of a cell sheet. 3T3 NIH fibroblasts were seeded on plasmonic substrates provided with the same specialized holders described above. A 1.5ml eppendorf was used with a glass slide (not coverslip). 1 x 10⁵ cells/well were added to each eppendorf vial and capped with loose laboratory film (Parafilm). The cells were incubated at 37 °C for 2 weeks replacing the medium with fresh culture medium every 3-4 days. After laser treatment, cells were left for 4 hrs and then, using light pipetting, a cell sheet was released from the gold surface. In order to take an image, the sheet was carefully transferred to a clean glass slide and a mosaic image (software available from Zeiss) taken using a x5 objective.

### B) Results

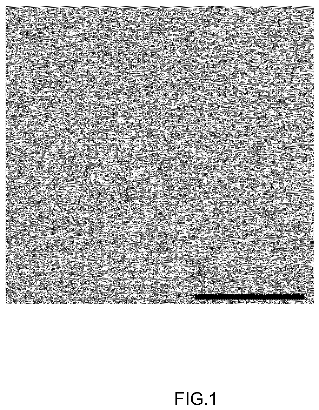

Au nanoparticles were deposited on a glass substrate by block co-polymer micellar nanolithography (BCMN), in which inverse micelles of the block copolymer poly-2-vinylpyridine (PS-b-P2VP) containing Au ions in the hydrophilic core were deposited by dip-coating onto a substrate, forming an ordered hexagonal array. The monolayer of inverse micelles was then subjected to a short treatment of oxygen plasma during 4 minutes, reducing the Au ions into Au nanoparticles with an average diameter of 15 nm, attached to the glass substrate. Note this short plasma treatment was essential to allow the subsequent growth steps as the adsorbed Au nanoparticles act as nuclei. A representative SEM image of the monolayer of small Au nanoparticles deposited by BCLM after a short plasma treatment of 4 minutes is shown in FIG. 1.

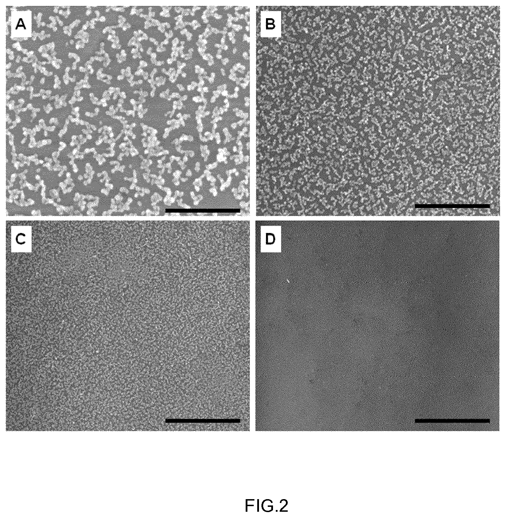

A longer plasma treatment would render an array of nanoparticles that are chemically inert to any further growth. Following this plasma treatment Au nanoparticles are partially embedded into the substrate, which greatly enhances the robustness of the nanoparticle layer, thus preventing uptake by cultured cells. A subsequent chemical growth treatment by soaking the substrates in a solution of Au ions, ascorbic acid (AA) as a reducing agent and hexadecyltrimethylammonium bromide (CTAB) as a surfactant for directing the growth gave an anisotropic, Au nanostructure, comprising nanoparticles of ca. 30-50 nm, typically with elongated branches pointing to random directions. The distribution of shapes and the different orientations, in addition to relatively short interparticle distances (less than 10 nm) led to a strong plasmon coupling. A second treatment with oxygen plasma of 45 minutes completely reduced any remaining Au ions and completely cleaned the surface of the substrates by removing organic residues. Representative SEM images of the plasmonic substrates after the plasma treatment of 4 minutes are shown in FIG. 2.

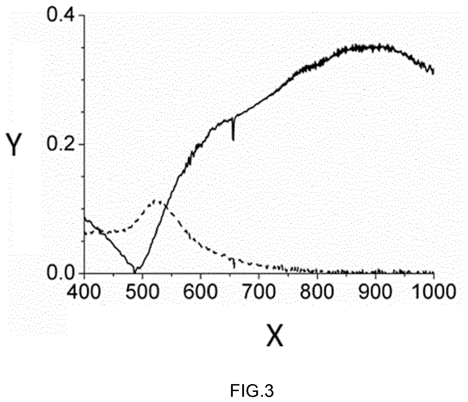

The plasmonic substrates exhibited extensive plasmon coupling, as can be seen in the vis-NIR spectra (FIG. 3). The absorbance band shifted from a single peak centered a ca. 525 nm, corresponding to a monolayer of Au nanoparticles before the growth step, to a broad band ranging from 600 to 1000 nm after growth and branching. Thus, the obtained plasmonic features were well suited for use in combination with NIR irradiation.

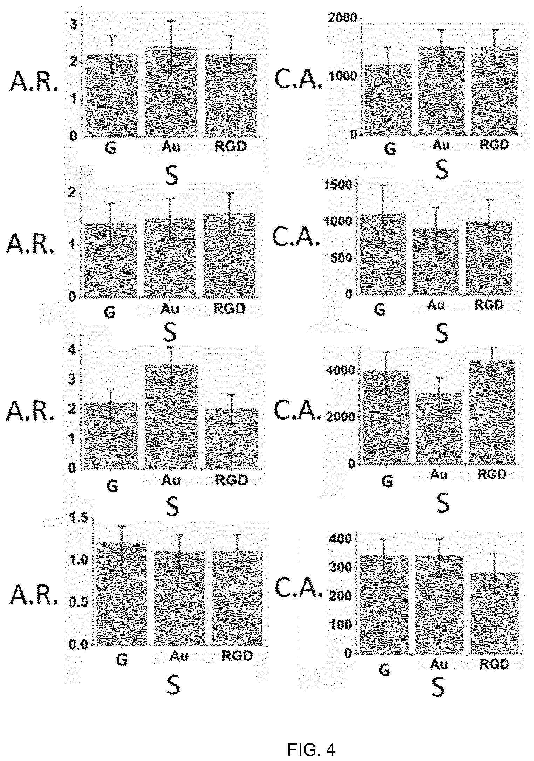

The plasmonic cell-culture substrates offered a bare Au surface, which was easy to functionalize by thiol chemistry. Thus, by including the cRGD biofunctional group on top of a metallic substrate by simple thiol chemistry, the morphology and intracellular signaling of integrin-expressing cells could be modified. Five different cell lines were grown in the plasmonic supports: HeLa and A549 (tumoral human cells), HUVEC (non-tumoral human cells), J774 (murine macrophage-like cell line) and 3T3 NIH fibroblasts. Quantitative results on the cell morphology following detachment from culture supports and upon attachment to the culture supports are shown in FIG. 4.

The morphology of the attached HeLa and A549 cells onto the substrates was not affected by adsorption on glass, bare plasmonic substrates or cRGD-coated plasmonic substrates, as indicated by no significant changes in the aspect ratio or area per cell. Interestingly, HUVEC cells displayed some degree of stretching when attached to bare plasmonic substrates as compared to glass substrates, with an increased aspect ratio but a slightly reduced total area per cell. However, when the HUVEC cells were grown on cRGD-functionalized plasmonic substrates, their morphology was again similar to those grown on glass. The changes in the morphology of the HUVEC cells by including the c-RGD peptide on the surface of the substrate can be rationalized in terms of the integrin content of these cells. Inventors can thus conclude that cellular morphology can be tuned through substrate functionalization, modifying the interplay between cell type and biological ligands. Therefore, the bare plasmonic substrates of the invention can be used for successful culture and growth of different cell lines, while upon biofunctionalization *via* thiol chemistry, the features of living cells could be adjusted. Since a multivalent potential for culturing different cell lines is most desirable for a generalized use of the plasmonic substrates, these experiments suggest the general capability of the structured gold nanoparticle substrate of the invention for culturing any type of cells.

By exploiting the extensive plasmon coupling property and the excellent cell growth capability of the structured gold nanoparticle cell-culture support of the invention, successful detachment of living cells by remote NIR irradiation could be realized. Irradiation of cells grown on the substrate of the invention with a NIR laser was found to lead to a highly efficient and nearly complete detachment. These results could be achieved even when additional layers of plasmonic supports or glass or plastic layers where interposed between the light source and the irradiated cell coated culture support. Note that control experiments performed on glass slides resulted in detachment values below 10%. When using the substrate of the invention, either with no functionalization or functionalized with the c-RGD peptide, detachment was nearly complete in all cases. Each cell line required a minimum irradiation time and power density to achieve this detachment, as detailed in Table 1. Note that the values of used power density was much lower than in other studies using visible light coupled to the plasmon of individual nanoparticles (as in the case of the references cited above), usually in the range of dozens of W/cm². This comparatively low power of applied NIR radiation was key for the large viability of detached cells using the procedure disclosed herein.

**Table 1. Experimental conditions of NIR laser irradiation carried out at 980 nm for detachment of cells from the substrate of the invention.**

| **Cell line** | **Power density (mW/cm²)** | **Exposure time (min)** |
|---|---|---|
| HeLa | 340 | 20 |
| A549 | 340 | 40 |
| HUVEC | 145 | 5 |
| J774 | 340 | 40 |
| 3T3 NIH fibroblasts sheet | 305 | 30 |

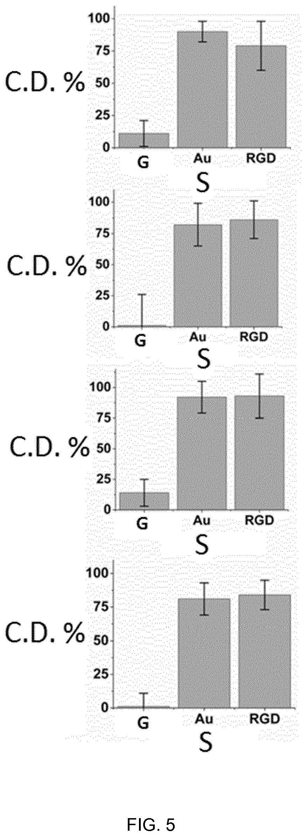

Note that functionalization with c-RGD did not affect detachment (FIG. 5), indicating that the effect was purely due to the Au nanoparticle substrate and not to receptor-integrin interactions occurring between cells and substrate.

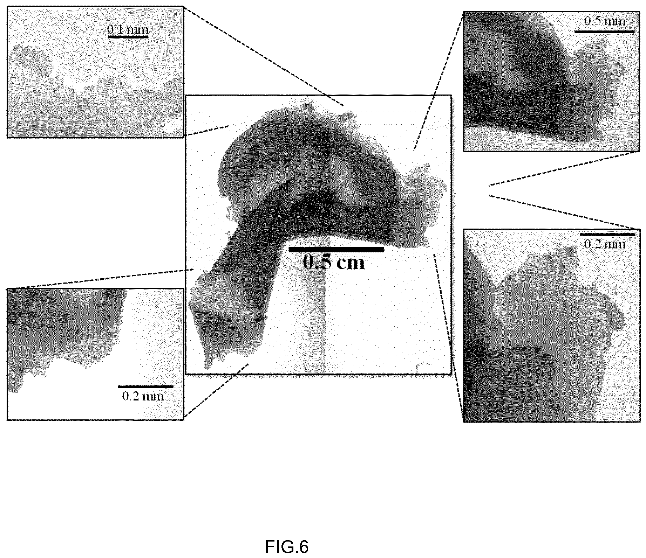

Inventors noted that the detachment efficiency of cells grown on the plasmonic adherent cell-culture support of the invention was related to the average cell-covered area. A549 and J774 cells displaying the smallest area per cell were detached with an efficiency of ca. 80 %. On the other hand, HeLa and HUVEC cells, which display a comparatively larger area per cell, exhibited detachment efficiencies approaching 100 %. Moreover, the irradiation time required for detachment was also shorter for HeLa and HUVEC cells than for A549 and J774 cells (Table 1). As this methodology for controlling cellular detachment from plasmonic substrates is of high interest for tissue engineering, inventors also studied the growth and detachment of 3T3 fibroblasts, a cell line that produces an interconnecting extracellular matrix (ECM) and is commonly used for tissue engineering related studies. 3T3 fibroblasts were grown over 2 weeks forming mono- or multi-layered cell sheets. By applying a similar power density and irradiation time as those used for HeLa cells, inventors observed detachment of the whole cell sheet, with dimensions above 500 µm, from the bare plasmonic substrate of the invention, see FIG. 6. Therefore, the concept of using plasmonic substrates for cell growth and detachment could be successfully applied, not only for single cells but also for cell sheets. These results suggest that the gold nanoparticle plasmonic support of the invention is a suitable technology for cell and tissue engineering applications.

Most importantly, since the use of the plasmonic substrates of the invention for cell detachment by NIR irradiation does not modify culture medium composition nor cell culture support properties once NIR irradiation has ceased, inventors realized that cells detached only from irradiated areas, allowing for the selective detachment of chosen cells and also for the re-attachment of cells on the support, i.e. reversible detachment.

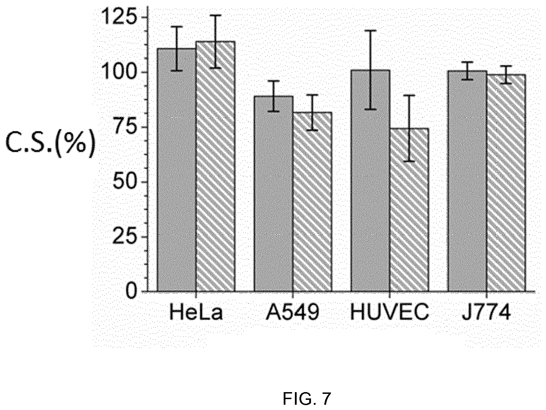

Lastly, once cells have been detached from a plasmonic substrate, an essential requirement toward subsequent use is cell viability. Inventors therefore performed MTT assays, which measured metabolic activity of the detached cells (see FIG. 7). For most cases, cell viability reached values of ca. 100%. Only in the case of A549 and HUVEC cells grown on RGD-functionalized substrates the viability was somewhat reduced, still up to values above 75%. Cell viability seemed to depend on the type of cell rather than on other experimental conditions used for detachment (e.g. irradiation time, intensity, etc.).

### REFERENCES CITED IN THE APPLICATION

Pasparakis G., et.al. "Laser-induced cell detachment and patterning with photodegradable polymer substrates" Angew. Chem. Int. Ed. 2011, vol. 50, pp. 4142-4145
Sada T., et.al. "Near-IR laser-triggered target cell collection using a carbon nanotube-based cell-culture substrate" ACS Nano 2011, vol. 6, pp. 4414-4421
Kolesnikova TA, et.al. "Laser-induced cell detachment, patterning, and regrowth on gold nanoparticle functionalized surfaces" ACS nano 2012, vol. 6, pp. 9585-9595
Zhu M., et.al. "Micropatterning thermoplasmonic gold nanoarrays to manipulate cell adhesion" ACS Nano 2012, vol. 6, pp. 7227-7233

## Claims

1. An adherent cell-culture support comprising gold nanoparticles on the surface where the cells attach to, wherein the size of the nanoparticles is from 5 nm to 2 microns, the distance between the nanoparticles is from 2 nm to 30 nm, and the anisotropy aspect ratio is greater than 1; wherein the nanoparticles are adsorbed onto the surface of the support which means that the nanoparticles are adhered to the surface of the support; wherein the nanoparticles are totally or partially embedded on the support; and wherein the shape of the nanoparticles is a grape cluster shape.

2. The adherent cell-culture support of claim 1, wherein the size of the nanoparticles is from 30 to 500 nm.

3. The adherent cell-culture support of any one of claims 1 to 2, further comprising thiolated molecules bound to the nanoparticles via their sulphur atoms.

4. The adherent cell-culture support of claim 3, wherein the thiolated molecules are selected from the group consisting of proteins, peptides, organic molecules, and nucleic acids.

5. A process for producing an adherent cell-culture support according to any one of claims 1 to 4 comprising gold nanoparticles on the surface where the cells attach to, comprising the steps of:
a) deposition of inverse micelles of a copolymer, comprising gold ions in the interior, onto the surface;
b) the result of step a) is treated with oxygen plasma for a period of from 2 to 4 minutes;
c) the result of step b) is treated twice with a solution of gold ions, a reducing agent and a surfactant, for at least 30 minutes each treatment;
d) the result of step c) is treated with oxygen plasma for a period of at least 10 minutes.

6. The process of claim 5, wherein the deposition in step a) is a deposition by dip-coating.

7. The process of any one of claims 5-6, wherein the copolymer in step a) is block-copolymer poly(styrene-b-2-vinylpyridine) and the reducing agent in step c) is ascorbic acid and the surfactant is cetyl trimethylammonium bromide (CTAB).

8. An adherent cell-culture support comprising gold nanoparticles on the surface where the cells attach to, as defined in any of the claims 1-4, obtainable by the process of anyone of claims 5-7.

9. A cell-culture device comprising the adherent cell-culture support of any one of claims 1 to 4 or 8.

10. A cell culture bioreactor comprising the cell-culture device of claim 9, and further comprising means to irradiate the cell-culture device of claim 9 with Near Infrared Light (NIR).

11. A method for culturing cells that comprises a step of seeding cells on the adherent cell-culture support of any one of claims 1-4 or 8.

12. The method of claim 11, further comprising a cell detachment step comprising an irradiation step of the cell loaded culture support with Near Infrared Light (NIR).

13. The method of claim 11, further comprising a step of cell detection comprising the detection of the Surface Enhanced Raman Spectroscopy signal emitted by the cell loaded culture support.

## Patentansprüche

1. Ein haftender Zellkulturträger umfassend Goldnanopartikeln auf der Oberfläche, an der die Zellen anhaften, wobei die Größe der Nanopartikeln von 5 nm bis 2 Mikrometern reicht, der Abstand zwischen den Nanopartikeln von 2 nm bis 30 nm reicht und das anisotropische Seitenverhältnis größer als 1 ist; wobei die Nanopartikeln auf der Oberfläche des Trägers adsorbiert sind, was bedeutet, dass die Nanopartikeln an der Oberfläche des Trägers angehaftet sind; wobei die Nanopartikeln vollständig oder teilweise auf dem Träger eingebettet sind; und wobei die Form der Nanopartikeln eine Traubenclusterform ist.

2. Der haftende Zellkulturträger des Anspruchs 1, wobei die Größe der Nanopartikeln von 30 bis 500 nm reicht.

3. Der haftende Zellkulturträger von einem der Ansprüche 1 bis 2, weiterhin umfassend thiolisierte Moleküle, die an die Nanopartikeln durch ihre Schwefelatome gebunden sind.

4. Der haftende Zellkulturträger des Anspruchs 3, wobei die thiolisierten Moleküle ausgewählt sind aus der Gruppe bestehend aus Proteinen, Peptiden, organischen Molekülen und Nukleinsäuren.

5. Ein Verfahren zur Herstellung von einem haftenden Zellkulturträger nach einem der Ansprüche 1 bis 4 umfassend Goldnanopartikeln auf der Oberfläche, an der die Zellen anhaften, umfassend folgende Schritte:
a) Abscheidung von inversen Mizellen eines Copolymers, mit darin enthaltenen Goldionen, auf die Oberfläche;
b) das Ergebnis des Schritts a) wird mit Sauerstoffplasma während eines Zeitraums von 2 bis 4 Minuten behandelt;
c) das Ergebnis des Schritts b) wird zwei Mal mit einer Lösung von Goldionen, einem Reduktionsmittel und einem Tensid während mindestens 30 Minuten für jede Behandlung behandelt;
d) das Ergebnis des Schritts c) wird mit Sauerstoffplasma während eines Zeitraums von mindestens 10 Minuten behandelt.

6. Das Verfahren des Anspruchs 5, wobei die Abscheidung im Schritt a) eine Abscheidung durch Tauchbeschichtung ist.

7. Das Verfahren von einem der Ansprüche 5-6, wobei das Copolymer im Schritt a) das Block-Copolymer Polystyrol-b-Poly(2-vinylpyridin) ist und das Reduktionsmittel im Schritt c) Ascorbinsäure ist und das Tensid Cetyltrimethylammoniumbromid (CTAB) ist.

8. Ein haftender Zellkulturenträger umfassend Goldnanopartikeln auf der Oberfläche, an der die Zellen anhaften, wie in einem der Ansprüche 1-4 definiert, der durch das Verfahren von einem der Ansprüche 5-7 erhalten werden kann.

9. Eine Zellkulturvorrichtung umfassend den haftenden Zellkulturträger von einem der Ansprüche 1 bis 4 oder 8.

10. Ein Zellkulturbioreaktor umfassend die Zellkulturvorrichtung des Anspruchs 9 und weiterhin umfassend ein Mittel zur Bestrahlung der Zellkulturvorrichtung des Anspruchs 9 mit Nahinfrarotlicht (NIR-Licht).

11. Ein Zellkulturverfahren, das einen Schritt umfasst, in dem Zellen auf dem haftenden Zellkulturträger von einem der Ansprüche 1-4 oder 8 ausgesät werden.

12. Das Verfahren des Anspruchs 11, weiterhin umfassend einen Zellabtrennungsschritt umfassend einen Schritt, in dem der mit Zellen geladenen Kulturträger mit Nahinfrarotlicht (NIR-Licht) bestrahlt wird.

13. Das Verfahren des Anspruchs 11, weiterhin umfassend einen Zellermittlungsschritt umfassend die Ermittlung des Signals von oberflächenverstärkter Raman-Spektroskopie, das vom mit Zellen geladenen Kulturträger ausgesendet wird.

## Revendications

1. Un support de culture cellulaire collant comprenant des nanoparticules d'or sur la surface à laquelle collent les cellules, dans lequel la taille des nanoparticules est de 5 nm à 2 micromètres, la distance entre les nanoparticules est de 2 nm à 30 nm, et le ratio d'aspect d'anisotropie est supérieur à 1 ; dans lequel les nanoparticules sont adsorbées sur la surface du support, ce qui signifie que les nanoparticules sont collées à la surface du support ; dans lequel les nanoparticules sont intégrées complètement ou en partie sur le support ; et dans lequel la forme des nanoparticules est une forme de grappe de raisin.

2. Le support de culture cellulaire collant de la revendication 1, dans lequel la taille des nanoparticules est de 30 à 500 nm.

3. Le support de culture cellulaire collant de l'une quelconque des revendications 1 à 2, comprenant en outre des molécules thiolées liées aux nanoparticules à travers leurs atomes de soufre.

4. Le support de culture cellulaire collant de la revendication 3, dans lequel les molécules thiolées sont choisies dans le groupe constitué de protéines, peptides, molécules organiques et acides nucléiques.

5. Un procédé de production d'un support de culture cellulaire collant selon l'une quelconque des revendications 1 à 4 comprenant des nanoparticules d'or sur la surface à laquelle collent les cellules, comprenant les étapes de :
a) déposition de micelles inverses d'un copolymère, comprenant des ions d'or dans son intérieur, sur la surface ;
b) le résultat de l'étape a) est traité avec du plasma d'oxygène pendant une période de 2 à 4 minutes ;
c) le résultat de l'étape b) est traité deux fois avec une solution d'ions d'or, un agent de réduction et un tensioactif, pendant au moins 30 minutes pour chaque traitement ;
d) le résultat de l'étape c) est traité avec du plasma d'oxygène pendant une période d'au moins 10 minutes.

6. Le procédé de la revendication 5, dans lequel la déposition de l'étape a) est une déposition effectuée moyennant enduction par trempage.

7. Le procédé de l'une quelconque des revendications 5-6, dans lequel le copolymère dans l'étape a) est le copolymère bloc poly(styrène-b-2-vinylpyridine) et l'agent de réduction dans l'étape c) est l'acide ascorbique et le tensioactif est le bromure de cetyl triméthylammonium (CTAB).

8. Un support de culture cellulaire collant comprenant des nanoparticules d'or sur la surface à laquelle collent les cellules, tel que défini dans l'une quelconque des revendications 1-4, qui peut être obtenu par le procédé de l'une quelconque des revendications 5-7.

9. Un dispositif de culture cellulaire comprenant le support de culture cellulaire collant de l'une quelconque des revendications 1 à 4 ou 8.

10. Un bioréacteur de culture cellulaire comprenant le dispositif de culture cellulaire de la revendication 9, et comprenant en outre un moyen d'irradiation du dispositif de culture cellulaire de la revendication 9 avec de la lumière proche infrarouge (NIR).

11. Un procédé de culture de cellules qui comprend une étape d'ensemencement de cellules sur le support de culture cellulaire collant de l'une quelconque des revendications 1-4 ou 8.

12. Le procédé de la revendication 11, comprenant en outre une étape de séparation de cellules comprenant une étape d'irradiation du support de culture chargé avec des cellules avec de la lumière proche infrarouge (NIR).

13. Le procédé de la revendication 11, comprenant en outre une étape de détection cellulaire comprenant la détection du signal de spectroscopie Raman exaltée de surface émis par le support de culture chargé avec des cellules.
